# EUROPEAN PATENT SPECIFICATION

(11) **EP 2 799 871 B1**
(45) Date of publication and mention of the grant of the patent: **13.10.2021**
(21) Application number: 12863706.3
(22) Date of filing: 14.12.2012
(51) Int. Cl.: B03C 1/28, G01N 33/543, G01N 37/00, G01N 33/553

(54) **SAMPLE ANALYSIS DEVICE AND SAMPLE ANALYSIS METHOD**
PROBENANALYSEVORRICHTUNG UND PROBENANALYSEVERFAHREN
DISPOSITIF D'ANALYSE D'ÉCHANTILLON ET PROCÉDÉ D'ANALYSE D'ÉCHANTILLON

(30) Priority: 28.12.2011 JP 2011287251
(43) Date of publication of application: 05.11.2014
(73) Proprietor: Hitachi High-Tech Corporation, Minato-ku Tokyo 105-6409 (JP)
(72) Inventor: INABA Toru, Hitachi-shi Ibaraki 319-1292 (JP); MATSUOKA Shinya, Hitachinaka-shi Ibaraki 312-8504 (JP); SAKAZUME Taku, Hitachinaka-shi Ibaraki 312-8504 (JP); YAMASHITA Yoshihiro, Hitachinaka-shi Ibaraki 312-8504 (JP); SHIMADA Masafumi, Hitachinaka-shi Ibaraki 312-8504 (JP)
(74) Representative: Mewburn Ellis LLP
(86) International application number: PCT/JP2012/082443
(87) International publication number: WO 2013/099648

(56) References cited:
- WO-A1-2011/155489
- JP-A- H10 300 752
- JP-A- H11 148 901
- JP-A- H11 242 032
- JP-A- 2005 528 584
- JP-A- 2006 046 950
- US-A- 5 993 740
- None

## Description

### Technical Field

The present invention relates to devices for analyzing a sample, specifically to a sample analysis device that uses antigen-antibody reaction.

### Background Art

The immunoassay is described first as a typical example of a sample analysis. The immunoassay is a test that uses specific antigen-antibody reaction to detect or measure antibodies or antigens in humor (e.g., plasma, serum, and urine) for disease or pathology diagnosis. ELISA (Enzyme-Linked Immunosorbent Assay) is a representative example of the immunoassay. In ELISA, an antibody (first antibody) against an antigen of interest is immobilized to the bottom of a container, and a sample such as plasma, serum, and urine is applied so the antigen in the sample binds to the first antibody. An antibody (second antibody) linked to a labeling reagent is then applied so it binds to the antigen bound to the first antibody. The signal produced by the label is then detected to determine the presence or absence of the antigen, or the amounts of the antigen in the sample. A fluorescent substance is used as the label, for example. In this case, the chromogenic reaction occurs more strongly in direct proportion to the number of the labeled second antibodies, i.e., the amounts of antigen, and the antigen in the sample can be quantified by detecting the luminescence of the fluorescent substance with a device such as a photomultiplier.

In a specific example of an ELISA immunoassay device, magnetic particles are used as the solid phase, and the first antibody is immobilized on the surfaces of the magnetic particles. The second antibody is linked to a substance (luminescent substance) labeled with a fluorescent dye. The antigen contained in a sample binds to the magnetic particles via the first antibody in response to an antigen-antibody reaction that occurs upon mixing a biological detection substance (antigen) with the magnetic particles immobilizing the first antibody. Upon reaction with the second antibody, the magnetic particles are bound to the luminescent substance via the second antibody, the antigen, and the first antibody. The amounts of the luminescent substance vary with the amounts of the detection substance contained in the sample, i.e., the amounts of the antigen.

The magnetic particles bound to the detection substance are captured at a specified location, and by using a laser, the luminescent substance bound to the magnetic particles emits luminescence. The luminescence intensity can then be detected to quantitatively determine the amounts of the detection substance, specifically the antigen amounts in the sample.

A high-sensitive immunoassay uses what is called B/F (bond/free) separation (separation of an antigen-antibody complex and free antibodies or antigens), in which the magnetic particles bound to the detection substance (antigen) are captured at a specified location with a magnet while displacing the solution containing antibodies not bound to the antigens. PTL 1 to PTL 4 describe methods of capturing magnetic particles at the predetermined position with an analysis device.

### Citation List

### Patent Literature

PTL 1: JP-A-8-62224
PTL 2: JP-A-11-242032
PTL 3: JP-A-7-248330
PTL 4: JP-T-2003-502670

US5993740 discloses arrangements in which magnetic particles are used as the solid phase to carry out immunoassay. In a reaction vessel, the magnetic particles are mixed with a sample containing TSH as an analyte and a labeled antibody. A chemiluminescent label material is bound onto the magnetic particles with an immunoreaction. A fluid including the above mixture is introduced to a chamber inside a flow through cell, the chamber being sized to have a width greater than a depth. The magnetic particles in the fluid are trapped by a magnet so as to be spread in the planar form over a predetermined area within the chamber, while useless materials are discharged outwardly of the chamber. The chamber is then filled with a buffer solution containing an attractant. By applying a voltage to electrodes disposed in the chamber, the label material on the magnetic particles is excited to emit an electro-chemiluminescence. The luminescence is detected by a photomultiplier.

WO2011/155489 discloses an analysis device and an analysis method which are described as preventing non-uniformities in the adhesion of magnetic particles and which detect with greater accuracy and precision. The disclosed sample analysis device is provided with a flow channel through which a sample containing magnetic particles flows, and a magnetic field generating means which generates a magnetic field for capturing the aforementioned magnetic particles in a magnetic particle capturing region in said flow channel. The disclosed sample analysis device is configured such that the cross-section area of the downstream end of the flow channel in the aforementioned magnetic particle capture region is greater than that of the upstream end thereof, and/or is configured such that the magnitude of the magnetic field generated by the aforementioned magnetic field generating means is greater downstream than upstream of the aforementioned magnetic particle capture region.

### Summary of Invention

### Technical Problem

Capturing of magnetic particles at the predetermined position involves the following problems. As described in the Background Art section, a laser and a detector are usually fixed at specified locations in an analysis device. This necessitates the magnetic particles to be captured at the predetermined position as determined by these locations. It is important that the magnetic particles are uniformly captured at such predetermined position to improve the measurement accuracy of the analysis device. However, this involves many problems, for example, as described below using PTL 1 (JP-A-8-62224), PTL 2 (JP-A-11-242032), PTL 3 (JP-A-7-248330), and PTL 4 (JP-T-2003-502670).

What often happens when B/F separation is performed with magnetic particles captured at the predetermined position is that the force exerted by the magnet (the force by which the magnetic particles are attracted to the magnet surface) becomes weaker in the vicinity of the side walls of the channel where the magnet ends are situated (as used herein, magnet ends are portions of the magnet other than the ends with the magnetic poles), with the result that the magnetic particles are captured more at the central portion of the channel, and less sufficiently in the vicinity of the side walls. The result is that the solution displaced in the B/F separation remains in the magnetic particle aggregate because of the interface tension of the solution, and B/F separation becomes insufficient.

The magnet force becomes weaker in the vicinity of the side walls of the channel where the magnet ends are situated also when, for example, fluorescence detection is performed for the magnetic particles captured at the predetermined position. In this case, the magnetic particles may not be sufficiently captured as above. This may result in poor luminescence sensitivity, and poor measurement performance.

The detection channels of the analysis devices described in PTL 1 and PTL 2 (JP-A-8-62224, JP-A-11-242032**)** have a uniform circular shape, or a uniform rectangular shape along the flow direction at the predetermined position where the magnetic particles are captured. On the other hand, the magnet width is smaller than the channel width, and undesirably creates a nonuniform capture distribution of magnetic particles.

In PTL 3 (JP-A-7-248330), the channel width and the magnet width are both 5 mm. This creates a poor magnetic particle capture distribution in the side walls of the channel, and lowers luminescence intensity.

In PTL 4 (JP-T-2003-502670), the magnetic particles are captured at the predetermined position under the magnetic field generated by an electromagnet, instead of a permanent magnet as used in PTL 1, PTL 2, and PTL 3. The electromagnet used to capture the magnetic particles is wider than the predetermined position in the flow direction. However, it is not preferable to increase the magnetic field wider than the magnetic particle-capturing predetermined position in the flow direction because it has the possibility of unnecessarily spreading the magnetic particles. This may lower luminescence intensity.

With the recent movement toward more accurate analysis, there is a greater demand for more uniformly capturing the magnetic particles throughout the predetermined position. This has created the need to increase the capture amounts in the vicinity of the channel side walls, taking into consideration the influence of the magnetic field gradient generated by the magnet, specifically by making the magnetic field gradient smaller in the vicinity of the channel side walls. Accordingly, there is a need for a detector with which the amounts of the magnetic particles captured at the side wall of the channel can be increased for more accurate analysis.

### Solution to Problem

According to a first aspect of the present invention there is provided a detector according to claim 1.

According to a second aspect of the present invention there is provided a sample analysis device according to claim 3.

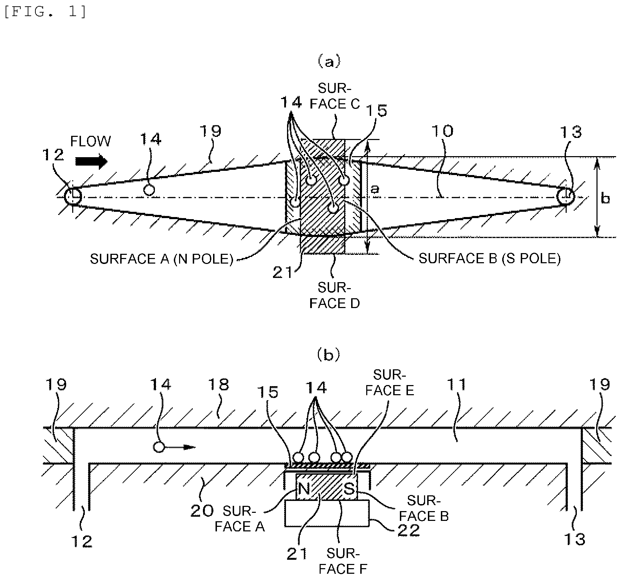

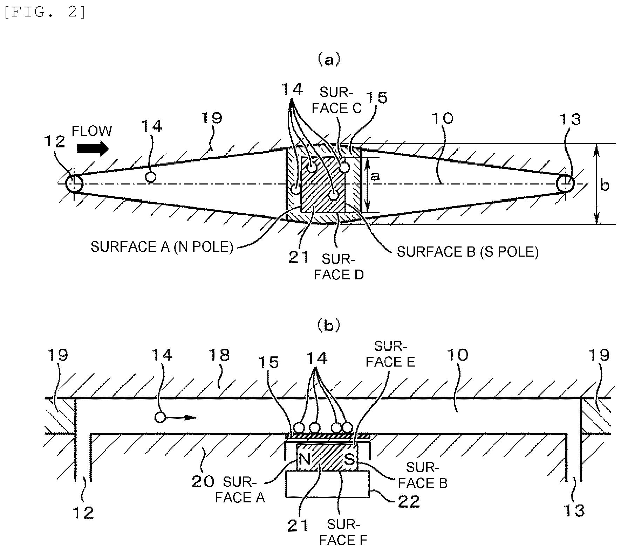

The present means is described below in detail. FIG. 2 represents the detection channel of a conventional sample analysis device. The magnet is disposed in such a manner that the surfaces (surfaces A and B) with the magnetic poles are perpendicular to the flow direction of the detection channel, and the surfaces (surfaces D and C) having no magnetic poles are parallel to the flow direction of the detection channel. Note that the surface capturing the magnetic particles is surface E, and the surface held by the slide mechanism is surface F. In the conventional detection channel, the width of the magnet particle-capturing magnet or electromagnet representing magnetic field generating means (the distance between surface D and surface C) is smaller than the channel width of the detection channel in the predetermined position where the magnetic particles are captured, and the magnetic particles cannot be captured in the vicinity of the channel side walls. On the other hand, in the detection channel as shown in FIG. 1, the width of the magnet particle-capturing magnet or electromagnet representing magnetic field generating means (the distance between surface D and surface C) is made wider than the channel width to capture the magnetic particles in the vicinity of the side wall of the channel. This increases the proportion of the magnet's attraction force acting on the magnetic particles that would otherwise remain uncaptured and flow out. The total number of the captured magnetic particles can thus increase, and the capture rate can improve.

### Advantage Effects of Invention

The foregoing means allows magnetic particles to be uniformly captured in the measurement region, and can provide improvements in, for example, B/F separation, washing efficiency, measurement accuracy, and reproducibility of the measurement result.

### Brief Description of Drawings

[FIG. 1] FIG. 1 shows a top view and a cross sectional view of the shapes of a detection channel and a magnet of the present invention.
[FIG. 2] FIG. 2 shows a top view and a cross sectional view of the shapes of a conventional detection channel and a conventional magnet.
[FIG. 3] FIG. 3 is a schematic diagram of an immunoassay device.
[FIG. 4] FIG. 4 is a magnified view of the detection channel.
[FIG. 5] FIG. 5 represents distributions of the force acting on magnetic particles in a measurement region in the present invention and the related art.
[FIG. 6] FIG. 6 is a flowchart representing a magnetic particle movement analysis.
[FIG. 7] FIG. 7 shows diagrams comparing the capture distributions in the measurement regions of the present invention and the related art.
[FIG. 8] FIG. 8 is a diagram representing the relationship between the magnet width-to-channel width ratio and the uniformity of a capture distribution.
[FIG. 9] FIG. 9 is a diagram comparing the measured luminescence between the proposed shape and the conventional shape.
[FIG. 10] FIG. 10 is a diagram representing the overall configuration of an analysis device.

### Description of Embodiments

An embodiment of the present invention is described below with reference to the accompanying drawings.

An immunoassay device as an example of a sample analysis device of the present embodiment is described first. The present invention is not limited to immunoassays, and is applicable to any sample analysis device, provided that it uses magnetic particles, and captures magnetic particles by switching magnetic field strengths. The technique of the present invention is also applicable to analytical devices used in the field of DNA, chemistry, and other applications.

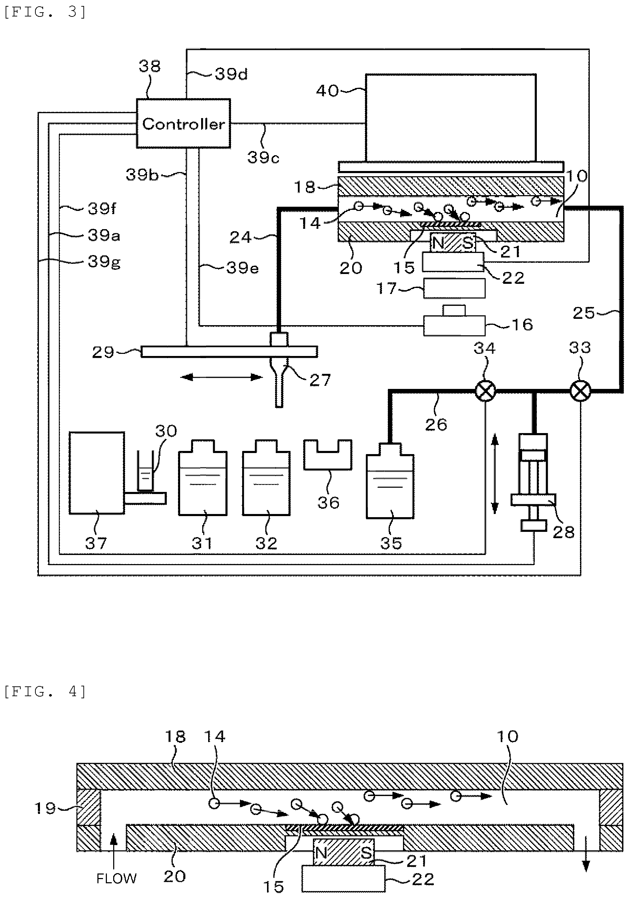

FIG. 3 represents a schematic structure of the immunoassay device. Referring to FIG. 3, a detection channel 10 is connected to a nozzle 27 and a pump 28 via a tube 24 and a tube 25. The nozzle 27 is movably installed on an arm 29, and a suspension container 30, a buffer container 31, and a washing liquid container 32 are installed within the movable range of the nozzle 27.

A valve 33 is provided on the tube 25 between the detection channel 10 and the pump 28. The pump 28 enables accurate volumes of liquid to be suctioned and ejected under the control of a controller 38 via a signal line 39a. The pump 28 is in communication with a waste liquid container 35 via a tube 26.

A detector has a detection channel window 18 and a detection channel base 20, both of which are made of transparent material. Inside the detector is the detection channel for flowing a solution. Because the whole channel is transparent, the detector passes light to allow an observer to see the state of the flow inside the channel. The side walls of the channel are not necessarily required to be formed of transparent material, and only the window for passing light may be made of transparent material.

The side walls of the transparent channel of the detector is preferably made of a material that is substantially transparent for the wavelength of the light emitted by the labeled substance of the magnetic particle complex captured in a measurement region inside the flow cell. Materials, for example, such as glass, quartz, and plastic are preferably used.

A laser light source 16, and a condensing lens 17 are installed in areas beneath the detection channel base 20. The laser emitted by the laser light source 16 is condensed through the condensing lens 17, and can irradiate a measurement region 15 in the detection channel 10.

The detector uses a magnet 21 (magnetic field applying means) used as a means to capture magnetic particles. The magnetic pole surfaces (surface A and surface B) of the magnet are perpendicular to the flow direction. Referring to FIG. 3, the magnet surfaces out of and into the plane of the paper are surfaces C and D, respectively. For capturing magnetic particles 14, the magnet 21 is moved to directly below the detection channel base 20. For example, the magnet 21 is installed on a slide mechanism 22 that is freely movable along the horizontal direction, and is moved to directly below the channel to capture magnetic particles. For washing the inside of the detection channel 10, the magnet 21 can be moved away from the detection channel 10 to sufficiently reduce its effect so that the detection channel 10 can be sufficiently washed. Here, the magnet 21 is described as being horizontally moved with the slide mechanism 22. However, the magnet 21 may be vertically moved, as long as the effect of the magnetic field of the magnet 21 can be sufficiently reduced for washing.

The controller 38 is connected to the arm 29, a photodetector 40, the slide mechanism 22, the laser light source 16, the pump 28, the valve 33, and a valve 34 to control these members.

The magnetic particles 14 spread in a planar fashion as they travel along the gradually widening path inside the detection channel 10, and are captured in the measurement region 15 under the magnetic force of the magnet 21. The measurement region also can be called capture region. For luminescence measurement, the magnet 21 cancels the magnetic force. Here, there is no liquid flow inside the detection channel 10, and the magnetic particles 14 remain captured in the measurement region 15, and stay inside the detection channel 10. Under no applied magnetic field to the detection channel 10, the laser light source 16 installed beneath the detection channel 10 emits a laser beam to the magnetic particles 14 remaining in the measurement region 15. The resulting luminescence from the labeled substance on the magnetic particles 14 can then be measured to determine the luminescence from the solid phase at high sensitivity.

The detection channel 10 is formed of a light transmissive material, specifically a material selected from high optical transmittance materials such as acryl. The photodetector 40 may be realized by, for example, a photomultiplier.

The detection channel 10 has a width that is 2 to 20 times its depth (thickness), so that the flow of the particles introduced with the fluid flow can easily spread laterally. Ideally, the magnetic particles preferably spread in the form of a single layer with respect to the detection channel 10. In actual practice, however, the particles may have some overlap, and form multiple layers under the influence of the magnetic field.

The capture distribution of the particles in the detection channel 10 is determined by the balance between the magnetic force of the magnetic field from the magnet 21 disposed on the lower side of the detection channel 10, and the drag created upon introduction of the suspension containing the reaction mixture. The magnetic field inside the detection channel 10 is preferably about 0.1 to 0.5 T. The accompanying liquid flow rate is preferably about 0.05 to 0.10 m/s. The flow rate needs to be appropriately selected, because particle separation occurs when the force created by the flow rate exceeds the force that captures the particles under the magnetic force.

The magnetic particles 14 are preferably any of the following particles.
(1) Paramagnetic, superparamagnetic, ferromagnetic, or ferrimagnetic particles
(2) Paramagnetic, superparamagnetic, ferromagnetic, or ferrimagnetic particles encapsulated in materials such as synthetic high molecular compounds (e.g., polystyrene, nylon), natural polymers (e.g., cellulose, agarose), and inorganic compounds (e.g., silica, glass).

The particle diameter of the magnetic particles 14 is preferably 0.01 µm to 200 µm, more preferably 1 µm to 10 µm. The specific gravity is preferably 1.3 to 1.5. Such magnetic particles 14 do not easily settle, and are easily suspended in the liquid. The magnetic particle surface is bound to a substance that has a specific binding property for the analyte, for example, an antibody having a specific binding property for antigen.

The labeled substance is preferably any of the following. The labeled substance is specifically bound to the analyte by using an appropriate means, and luminescence is produced by using an appropriate means.

(1) Labeled substance used for fluorescent immunoassays . For example, an antibody labeled with fluorescein isothiocyanate.
(2) Labeled substance used for chemiluminescent immunoassays. For example, an antibody labeled with acridinium ester.
(3) Labeled substance used for chemiluminescent enzyme immunoassays. For example, an antibody labeled with a chemiluminescent enzyme that uses luminol or adamantyl derivatives as a substrate.

The sample analyzed is a sample of biological fluid origin, for example, such as serum and urine. When the sample is a serum, examples of the analyzed components include various tumor markers, antibodies, antigen-antibody complex, and single protein. Here, the specific component is TSH (thyroid hormone).

The suspension container 30 stores a sample mixture prepared in advance by mixing the analyte sample with a beads solution, a first reagent, a second reagent, and a buffer, and reacting the mixture for a certain time period at a certain temperature (37°C).

The beads solution is a solution obtained by dispersing the magnetic particles 14 in a buffer after embedding a particulate magnetic substance in a matrix such as polystyrene. The matrix surface is bound to streptavidin that can bind to biotin.

The washing liquid container 32 stores a washing liquid used to wash inside of the detection channel 10 and the tube 24.

The operation of the present embodiment is described below. One cycle of analysis consists of a suspension suction period, a particle capture period, a detection period, a washing period, a reset period, and a preliminary suction period. A cycle begins upon setting the suspension container 30 in the predetermined position after the suspension processed in a reaction unit 37 is stored in the suspension container 30.

In the suspension suction period, the slide mechanism 22 comes into operation in response to the received signal from the controller 38, and moves the magnet 21 to below the detection channel 10. Here, the valve 33 is open, and the valve 34 is closed. The arm 29 comes into operation in response to the received signal from the controller 38, and inserts the nozzle 27 into the suspension container 30. The pump 28 then starts a certain suction operation upon receiving a signal from the controller 38. In response, the suspension in the suspension container 30 enters the tube 24 via the nozzle 27. The pump 28 is arrested in this state, and the arm 29 is operated to insert the nozzle 27 into a washing mechanism 36. The nozzle tip is washed as it passes through the washing mechanism 36.

In the particle capture period, the pump 28 creates suction at a certain rate in response to the received signal from the controller 38, drawing the suspension inside the tube 24 into and through the detection channel 10. Because of the magnetic field created by the magnet 21 in the detection channel 10, the magnetic particles 14 contained in the suspension are drawn toward the magnet 21, and captured to the surface in the measurement region 15.

In the detection period, the slide mechanism 22 comes into operation, and the magnet 21 is moved away from the detection channel 10. The laser light source 16 then emits a laser beam in response to the received signal from the controller 38, and irradiates the measurement region 15 through the condensing lens 17. The fluorescent dye bound to the magnetic particles 14 in the measurement region 15 exhibits luminescence. A fluorescence filter cuts off certain wavelengths, and the selected wavelength is detected by the photodetector 40 realized by, for example, a CCD camera, or a photomultiplier. The photodetector 40 detects the luminescence intensity, and sends the detected signal to the controller 38. The laser is turned off after a certain time period. While in the detection period, the arm 29 operates to insert the nozzle 27 into the washing mechanism 36.

In the washing period, the pump 28 creates suction to draw the washing liquid out of the washing liquid container 32 into and through the detection channel 10. Here, because the magnetic field is away from the detection channel 10, the magnetic particles 14 do not stay on the measurement region 15, and flow out with the buffer.

In the reset period, the valve 33 is closed, and the valve 34 is opened for the ejection operation of the pump 28. The liquid in the pump 28 discharges to the waste liquid container 35.

In the preliminary suction period, the buffer is suctioned to fill the tube 24 and the detection channel 10 with the buffer. The next cycle is ready to begin after the preliminary suction period.

FIG. 1 represents what is considered to be the best relative positions of the detection channel 10 and the magnet 21 in the present embodiment. For comparison with FIG. 1, FIG. 2 represents the conventional relative positions of the detection channel 10 and the magnet 21. Referring to FIGS. 1 and 2, the magnet is disposed in such a manner that the end surfaces (surfaces A and B) with the magnetic poles are perpendicular to the direction of the flow in the detection channel, and that the end surfaces (surfaces D and C) having no magnetic poles are parallel to the direction of the flow in the detection channel. Note that the surface capturing the magnetic particles is surface E, and the surface held by the slide mechanism is surface F. Referring to FIG. 2, the relative positions of the detection channel 10 and the magnet 21 are such that the width a (the distance between surface D and surface C) of the magnet is equal to or smaller than the channel width b of the measurement region (also referred to as the capture region of the magnetic particles) . On the other hand, referring to FIG. 1, the width a of the magnet 21 is greater than the channel width b.

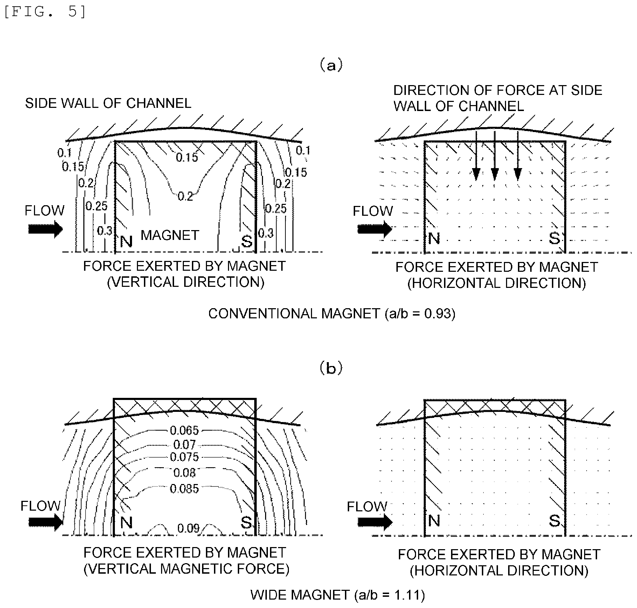

The following describes how the magnet width of the capture region affects the capturing of the magnetic particles . FIG. 5 represents the force exerted by the magnet on the magnetic particles in the capture region as calculated from the magnetic moment on the magnetic field and the magnetic particles using general-purpose magnetic field analysis software. In FIG. 5(a), the ratio (a/b) of magnet width a and channel width b is 0.93 (the magnet width is smaller than the channel width). In FIG. 5(b), the ratio a/b is 1.11 (the magnet width is greater than the channel width) . In these diagrams, the vertical component of the force exerted by the magnet is represented by a contour diagram (representing the force acting to capture the magnetic particles in a direction perpendicular to surface E), and the horizontal component is represented by a vector diagram (representing the force acting on the magnetic particles in a direction parallel to surface E). The channel and the magnet are also shown to clarify the relative positions of these members with regard to the channel width and the magnet width. Since these diagrams are symmetrical about the horizontal axis, only the top half is shown.

When the ratio a/b of magnet width and channel width is 0.93 as in FIG. 5(a), the horizontal component of the force is directed toward the center of the channel in the vicinity of the side walls of the channel where the surface D or C of the magnet are situated, and the vertical component of the force acting to capture the magnetic particles to surface E is small, as shown in the contour diagram representing the vertical force. It was also found that the horizontal component of the force acting on the magnetic particles is directed toward the center of the channel in the vicinity of the channel side walls. As demonstrated above, the force acts on the magnetic particles more strongly in the horizontal direction toward the center of the channel than in the vertical direction in the vicinity of the channel side walls, and the magnetic particles are not captured as easily in the vicinity of the channel side walls, and tend to accumulate in layers near the center of the channel.

On the other hand, when the magnet width is larger than the channel width as in FIG. 5(b), the force hardly acts in the horizontal direction as compared to FIG. 5(a). This is because the surfaces D and C of the magnet are more distant away from the vicinity of the side wall of the channel, creating a more gradual magnetic field gradient (smaller magnetic field changes), and reducing the horizontal component of the force that acts on the magnetic particles in the vicinity of the channel side walls. The magnetic particles can thus be more uniformly captured in the vicinity of the side walls and the center of the channel than in FIG. 5(a).

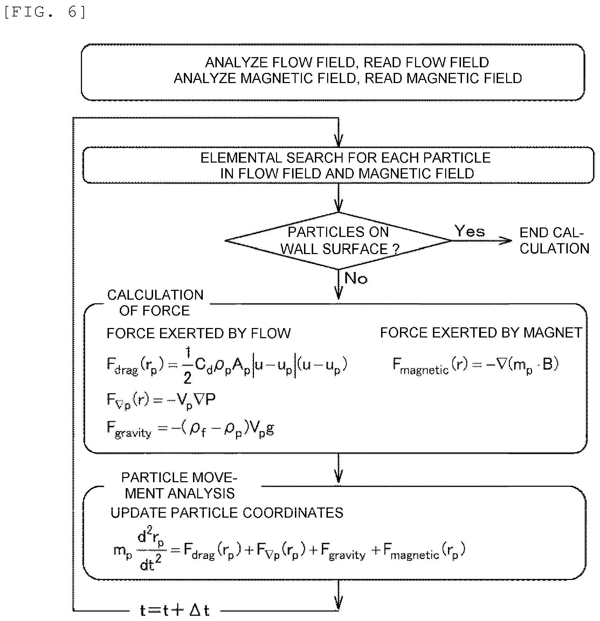

On the basis of these findings, a movement analysis of magnetic particles was conducted to examine the effect of making the magnet width wider than the channel width. FIG. 6 represents a flowchart of the movement analysis. The analysis is performed for the flow field inside the channel flowing the magnetic particles, using general-purpose fluid analysis software. Simultaneously, the magnetic field generated by the magnet is analyzed with general-purpose magnetic field analysis software. The states of these fields are used to analyze the forces acting on the magnetic particles, specifically, the force exerted by the flow, the force due to the pressure gradient of the flow, the buoyancy force acting on the particles, and the force exerted by the magnet. The movement of the magnetic particles can then be analyzed by solving an equation of motion for each particle in small increments of time, taking into equation these various external forces acting on each magnetic particle.

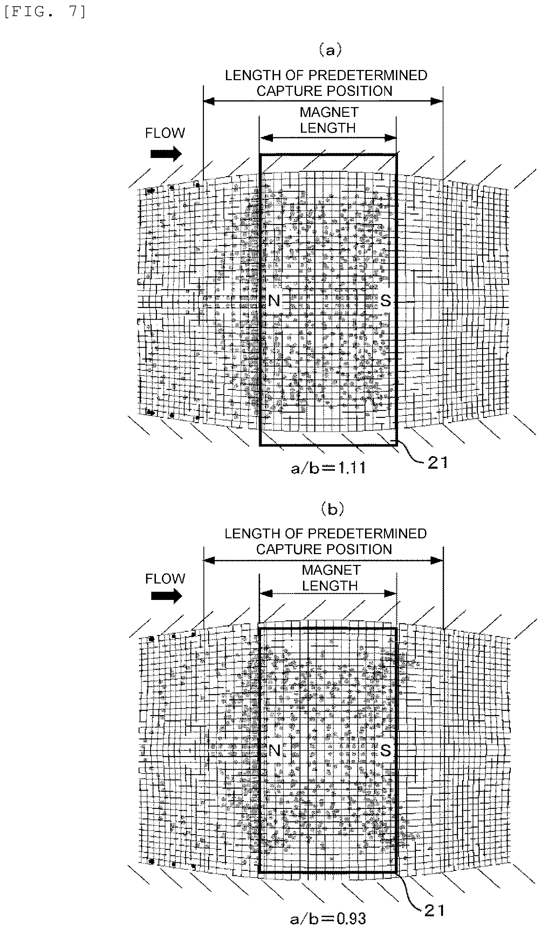

FIG. 7(a) and (b) represent the results of the analysis of the capture distribution of the magnetic particles when the ratio a/b of magnet width a and channel width b is 1.11 (FIG. 7(a)) and 0.93 (FIG. 7(b)). It can be seen that more magnetic particles are captured in the vicinity of the channel side walls when the magnet width is greater than the channel width. This is because the wider magnet width than the channel width decreases the horizontal component of the force that acts on the magnetic particles in the vicinity of the channel side walls, as described in FIG. 5. The magnetic particles are also captured more uniformly throughout the channel width as compared to FIG. 7(b).

The best mode of the magnet width is that the magnet width is wider than the channel width over the whole region. It is not difficult to imagine that more magnetic particles will be captured, and the luminescence intensity will increase even when the magnet width is increased wider than the channel width only in a part of the region. According to an example not covered by the appended claims, the surface E of the magnet may be trapezoidal in shape, and may partially extend beyond the side walls of the channel in the capture region.

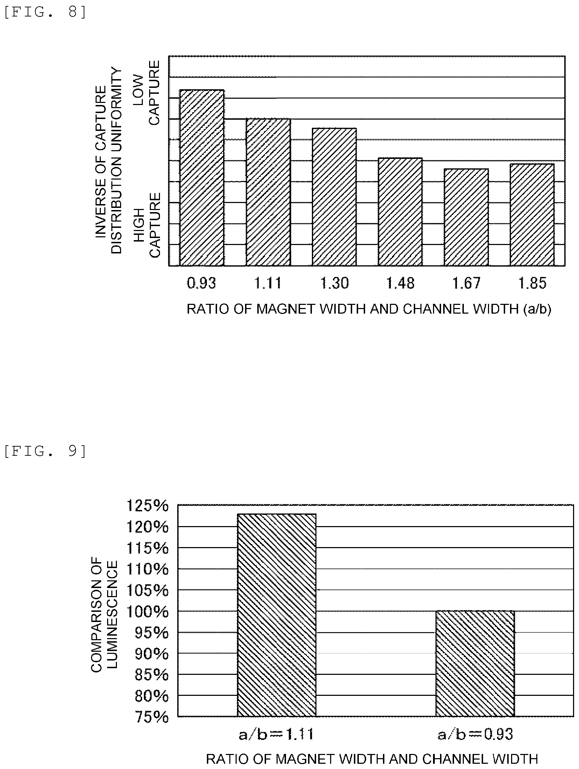

The effect of magnet width on capture distribution was systematically examined. FIG. 8 represents the uniformity of capture distribution examined with gradually increasing magnet widths relative to a constant channel width using a magnetic particle movement program. The horizontal axis in the graph represents the ratio of magnet width to channel width. The ratio increases as the magnet width increases relative to the channel width. The vertical axis is the inverse of capture distribution uniformity. Smaller values mean that the magnetic particles are more uniformly captured.

It can be seen that the uniformity of capture distribution in the measurement region improves as the ratio a/b of magnet width a and channel width b increases. The effect can be obtained as long as the magnet width is wider than at least the channel width, and the optimum effect can be obtained when the ratio a/b of magnet width and channel width is about 1.67. As can be seen in the graph, increasing the magnet width further beyond this ratio causes poor uniformity in the capture distribution. The result suggests that there are optimal values for the magnet width.

The analysis suggested that more magnetic particles can be captured in the vicinity of the channel side walls when the magnet width a and the channel width b are 6.0 mm and 5.4 mm, respectively. Typically, the slope of the magnetic field distribution in the vicinity of the ends of the magnet does not have large effects with regard to size. Accordingly, effects can be obtained when the magnet width a is wider than the channel width b by at least 0.6 mm.

The analysis result that the uniformity improves with the wider magnet width than the channel width was tested with a test magnet used in the actual device.

FIG. 9 represents the result of luminescence intensity measurement at a/b of 0.93 and 1.11. As can be seen in the graph, the luminescence improves 23% by increasing the magnet width wider than the channel width. As demonstrated above, the magnetic particles can be uniformly captured by the magnet, and the luminescence can be improved by increasing the magnet width wider than the channel width. Further, because of the uniform capture distribution, improvements can be expected in, for example, B/F separation performance, washing efficiency, measurement accuracy, and measurement result reproducibility.

The embodiment represented in FIG. 1 was described through the case where the channel width varies along the flow direction. Specifically, the magnetic particles can be captured in the vicinity of the channel side walls, and a uniform capture distribution can be obtained when the magnet width (the distance between surface D and surface C) is wider than the channel width over the whole channel width of the measurement region. In an example not covered by the claims, improved uniformity also can be expected when the magnet width is wider only in a part of the region instead of the whole region, though the effect will be limited.

The idea concerning the magnet length and the capture region length in the flow direction is described below.

The capture distribution of magnetic particles spreads when the magnet length (the distance between surface A and surface B) is greater than the measurement region. Specifically, the magnetic particles are captured also outside of the region intended for capturing the magnetic particles. It is not difficult to imagine that this will lower the luminescence intensity. The magnet length thus needs to be smaller than the measurement region.

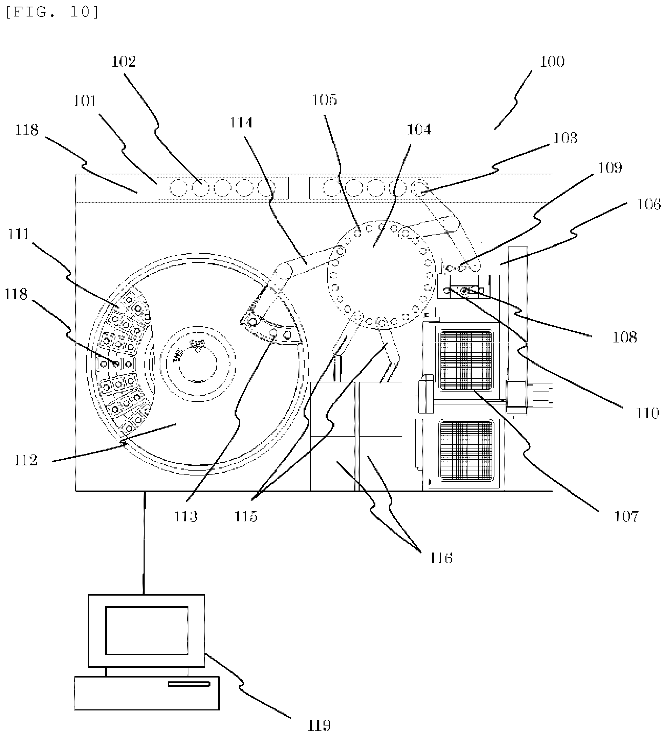

FIG. 10 represents an example of the actual implementation of the present embodiment as an automated immunoassay device.

A controller 119 of an assay device 100 creates an analysis plan upon receipt of a measurement request from an operator, and controls the operation of each mechanism by following the plan.

A sample container 102 for holding samples is installed in a rack 101 of the assay device 100, and a rack transport line 117 moves the sample container 102 to a sample dispensing position in the vicinity of a sample dispensing nozzle 103. A plurality of reaction vessels 105 is installable in an incubator disc 104. The incubator disc 104 is capable of rotational motion that moves the reaction vessels 105 installed along the circumferential direction to predetermined positions, including, for example, reaction vessel installation position, reagent ejection position, sample ejection position, detection position, and reaction vessel discarding position. A sample dispensing tip and reaction vessel transport mechanism 106 is movable in three directions along the X, Y, and Z axes, and transports a sample dispensing tip and a reaction vessel by moving over the range covering a sample dispensing tip and reaction vessel holding member 107, a reaction vessel mixing mechanism 108, a sample dispensing tip and reaction vessel discarding hole 109, a sample dispensing tip attaching position 110, and a predetermined position of the incubator disc 104.

A plurality of unused reaction vessels, and a plurality of unused sample dispensing tips are installed in the sample dispensing tip and reaction vessel holding member 107. The sample dispensing tip and reaction vessel transport mechanism 106 moves to above the sample dispensing tip and reaction vessel holding member 107, and lifts down to pick up one of the unused reaction vessels. After lifting up, the sample dispensing tip and reaction vessel transport mechanism 106 moves to above the reaction vessel installation position of the incubator disc 104, and lifts down to install the reaction vessel.

A plurality of reagent vessels 118 with reagents and diluting solutions is installed in a reagent disc 111. A reagent disc cover 112 is provided above the reagent disc 111 to maintain a predetermined temperature inside the reagent disc 111. A reagent disc cover opening 113 is provided in a portion of the reagent disc cover 112. A reagent dispensing nozzle 114 is capable of rotation and vertical motion. By undergoing rotation, the reagent dispensing nozzle 114 moves to above the opening 113 of the reagent disc cover 112, and lifts down to contact the tip of the reagent dispensing nozzle 114 to the reagent or diluting solution contained in the predetermined reagent vessel. The reagent or diluting solution is then suctioned into the reagent dispensing nozzle 114 in a predetermined amount. After lifting up, the reagent dispensing nozzle 114 moves to above the reagent ejection position of the incubator disc 104, and ejects the reagent or diluting solution into one of the reaction vessels 105.

The sample dispensing tip and reaction vessel transport mechanism 106 then moves to above the sample dispensing tip and reaction vessel holding member 107, and lifts down to pick up one of the unused sample dispensing tips. After lifting up, the sample dispensing tip and reaction vessel transport mechanism 106 moves to above the sample dispensing tip attaching position 110, and lifts down to install the sample dispensing tip. The sample dispensing nozzle 103 is capable of rotation and vertical motion. The sample dispensing nozzle 103 moves to above the sample dispensing tip attaching position 110, and lifts down to install the sample dispensing tip to the tip of the sample dispensing nozzle 103. The sample dispensing nozzle 103 with the installed sample dispensing tip moves to above the sample container 102 mounted on the transport rack 101, and lifts down to draw a predetermined amount of the sample held in the sample container 102. The sample dispensing nozzle 103 with the sample moves to the sample ejection position of the incubator disc 104, and lifts down to eject the sample into the reaction vessel 105 on the incubator disc 104 into which the reagent was dispensed. After ejecting the sample, the sample dispensing nozzle 103 moves to above the sample dispensing tip and reaction vessel discarding hole 109, and discards the used sample dispensing tip into the discarding hole.

The reaction vessel 105 with the ejected sample and reagent is moved to the reaction vessel transport position by the rotation of the incubator disc 104, and transported to the reaction vessel mixing mechanism 108 by the sample dispensing tip and reaction vessel transport mechanism 106. The reaction vessel mixing mechanism 108 rotates the reaction vessel to mix the sample and the reagent in the reaction vessel. The agitated reaction vessel is transported back to the reaction vessel transport position of the incubator disc 104 by the sample dispensing tip and reaction vessel transport mechanism 106. A reaction liquid suction nozzle 115 is capable of rotation and vertical motion. The reaction liquid suction nozzle 115 moves to above the reaction vessel 105 resting on the incubator disc 104 for a predetermined time period after the sample and the reagent were mixed. The reaction liquid suction nozzle 115 then lifts down, and draws the reaction mixture out of the reaction vessel 105. The reaction mixture drawn into the reaction liquid suction nozzle 115 is sent to a detector unit 116, and the subject of measurement is detected. The controller 119 then outputs and displays the measurement result based on the detection value of the subject. The reaction vessel 105 with the reaction mixture is moved to the reaction vessel discarding position by the rotation of the incubator disc 104. The sample dispensing tip and reaction vessel transport mechanism 106 then moves the reaction vessel 105 from the incubator disc 105 to above the sample dispensing tip and reaction vessel discarding hole 109, and the reaction vessel 105 is discarded through the discarding hole.

The present embodiment enables the magnetic particles to be uniformly captured, and can improve the accuracy and reproducibility of an analysis in an automated immunoassay device.

### Reference Sign List

10 Detection channel
12 Inlet of detection channel
13 Outlet of detection channel
14 Magnetic particle
15 Measurement region
16 Laser light source
17 Condensing lens
18 Detection channel window
19 Side wall of detection channel
20 Base of detection channel
21 Magnet
22 Slide mechanism
24, 25, 26 Tube
27 Nozzle
28 Pump
29 Arm
30 Suspension container
31 Buffer container
32 Washing liquid container
33, 34 Valve
35 Waste liquid container
36 Washing mechanism
37 Reaction unit
38 Controller
39 Signal line
40 Photodetector
100 Assay device
101 Rack
102 Sample container
103 Sample dispensing nozzle
104 Incubator disc
105 Reaction vessel
106 Sample dispensing tip and reaction vessel mixing transport mechanism
107 Sample dispensing tip and reaction vessel holding member
108 Reaction vessel mixing mechanism
109 Sample dispensing tip and reaction vessel discarding hole
110 Sample dispensing tip attaching position
111 Reagent disc
112 Reagent disc cover opening
114 Reagent dispensing nozzle
115 Reaction liquid suction nozzle
116 Detection unit
117 Rack transport line
118 Reagent vessel
119 Controller

## Claims

1. A detector comprising:
a channel (10) for flowing a sample liquid; and
magnetic field generating means (21), which is movable to directly below the channel (10), by which a magnetic particle (14) bound to a specific substance in the sample liquid is captured by magnetic attraction between the magnetic particle (14) and the magnetic field generating means (21) in a measurement region (15) of the channel (10),
wherein the magnetic field generating means (21) is disposed with two surfaces (A,B) having magnetic poles being perpendicular to the direction of the flow in the channel, and two surfaces (C,D) having no magnetic poles being parallel to the direction of the flow in the channel,
wherein the magnetic field generating means (21) is movable away from the channel (10) so that no magnetic field is applied to the channel (10), the detector further comprising a laser light source (16) that is configured to illuminate the magnetic particle (14) remaining in the measurement region (15) when no magnetic field is applied to the channel (10),
wherein the channel (10) is configured so that the magnetic particles (14) spread in a planar fashion as they travel along a gradually widening path inside the detection channel until they are captured in the measurement region (15) under the magnetic force of the magnetic field generation means (21);
the channel (10) having a maximum channel width b at the measurement region (15) in a direction perpendicular to the flow direction of the channel (10);
**characterized in that**
the width a of the magnetic field generating means (21), which is the distance between the surfaces (C,D) having no magnetic poles, in the direction perpendicular to the flow direction of the channel (10), and the maximum channel width b of the channel (10) at the measurement region (15) in the direction perpendicular to the flow direction of the channel (10), satisfy the ratio *a*/*b* of 1.11 ≤ *a*/*b* ≤ 1.85.

2. The detector according to claim 1, wherein the width a is greater than the width b by at least 0.6 mm.

3. A sample analysis device comprising the detector according to claim 1 or claim 2, wherein:
the channel (10) is a detection channel (10) through which the sample liquid containing the specific substance and the magnetic particle (14) bound to the specific substance is introduced to the measurement region (15);
the magnetic field generating means (21) is positioned outside of the detection channel (10) and in the vicinity of the measurement region (15) so as to capture the magnetic particle (14) in the measurement region (15) after the magnetic particle (14) is introduced into the detection channel (10); and
the sample analysis device further comprises:
supplying means that supplies the sample liquid to the detection channel (10);
measuring means that measures the specific substance captured in the measurement region (15); and
discharge means that discharges the magnetic particle (14) out of the detection channel (10) after the measurement.

4. The sample analysis device according to claim 3, wherein:
the detection channel (10) has an inlet (12) through which the supplying means introduces the sample liquid, and an outlet (13) through which the discharge means discharges the sample liquid after the measurement.

## Patentansprüche

1. Detektor, umfassend:
einen Kanal (10) zum Strömenlassen einer flüssigen Probe; und
ein Magnetfelderzeugungsmittel (21), das direkt an eine Position unterhalb des Kanals (10) bewegbar ist und durch das ein magnetisches Teilchen (14), das an eine spezifische Substanz in der flüssigen Probe gebunden ist, durch magnetische Anziehung zwischen dem magnetischen Teilchen (14) und dem Magnetfelderzeugungsmittel (21) in einem Messbereich (15) des Kanals (10) eingefangen wird,
wobei das Magnetfelderzeugungsmittel (21) mit zwei, magnetische Pole aufweisenden Flächen (A,B), die orthogonal zur Strömungsrichtung im Kanal sind, und zwei Flächen (C,D) ohne magnetische Pole, die parallel zur Strömungsrichtung im Kanal sind, versehen ist,
wobei das Magnetfelderzeugungsmittel (21) vom Kanal (10) wegbewegbar ist, sodass kein Magnetfeld an den Kanal (10) angelegt wird, wobei der Detektor weiters eine Laserlichtquelle (16) umfasst, die konfiguriert ist, um das magnetische Teilchen (14) zu beleuchten, das im Messbereich (15) verbleibt, wenn kein Magnetfeld an den Kanal (10) angelegt wird,
wobei der Kanal (10) so konfiguriert ist, dass die magnetischen Teilchen (14) sich auf planare Weise ausbreiten, während sie sich entlang eines sich allmählich erweiternden Pfads innerhalb des Detektionskanals fortbewegen, bis sie im Messbereich (15) durch die Magnetkraft des Magnetfelderzeugungsmittels (21) eingefangen werden;
wobei der Kanal (10) eine maximale Kanalbreite b im Messbereich (15) in einer Richtung orthogonal zur Strömungsrichtung des Kanals (10) aufweist;
**dadurch gekennzeichnet, dass**
die Breite *a* des Magnetfelderzeugungsmittels (21), bei der es sich um den Abstand zwischen den Flächen (C,D) ohne Magnetpole in der Richtung orthogonal zur Strömungsrichtung des Kanals (10) handelt, und die maximale Kanalbreite *b* des Kanals (10) im Messbereich (15) in der Richtung orthogonal zur Strömungsrichtung des Kanals (10) das Verhältnis *a*/*b* von 1,11 ≤ *a*/*b* ≤ 1,85 erfüllen.

2. Detektor nach Anspruch 1, wobei die Breite a um zumindest 0,6 mm größer ist als die Breite b.

3. Probenanalysevorrichtung, die den Detektor nach Anspruch 1 oder Anspruch 2 umfasst, wobei:
der Kanal ein Detektionskanal ist, durch den die flüssige Probe, welche die spezifische Substanz und das magnetische Teilchen (14) an die spezifische Substanz gebunden umfasst, in den Messbereich (15) eingebracht wird;
das Magnetfelderzeugungsmittel (21) außerhalb des Detektionskanals und in der Nähe des Messbereichs (15) positioniert ist, um das magnetische Teilchen (14) im Messbereich (15) einzufangen, nachdem das magnetische Teilchen (14) in den Detektionskanal eingebracht wurde; und
die Probenanalysevorrichtung weiters Folgendes umfasst:
ein Zufuhrmittel, das die Probenflüssigkeiten zum Detektionskanal zuführt;
ein Messmittel, das die im Messbereich (15) eingefangene spezifische Substanz misst; und
ein Ableitmittel, welches das magnetische Teilchen (14) nach der Messung aus dem Detektionskanal ableitet.

4. Probenanalysevorrichtung nach Anspruch 3, wobei:
der Detektionskanal einen Einlass (12), durch den das Zufuhrmittel die Probenflüssigkeit zuführt, und einen Auslass (13) aufweist, durch den das Ableitmittel die Probenflüssigkeit nach der Messung ableitet.

## Revendications

1. Détecteur comprenant :
un canal (10) pour faire s'écouler un liquide échantillon ; et
un moyen de génération de champ magnétique (21), qui est mobile pour être directement au-dessous du canal (10), à l'aide duquel une particule magnétique (14) liée à une substance spécifique dans le liquide échantillon est capturée par attraction magnétique entre la particule magnétique (14) et le moyen de génération de champ magnétique (21) dans une région de mesure (15) du canal (10),
dans lequel le moyen de génération de champ magnétique (21) est disposé avec deux surfaces (A, B) ayant des pôles magnétiques qui sont perpendiculaires à la direction de l'écoulement dans le canal, et deux surfaces (C, D) n'ayant pas de pôles magnétiques qui sont parallèles à la direction de l'écoulement dans le canal,
dans lequel le moyen de génération de champ magnétique (21) peut se déplacer au loin du canal (10) de telle sorte qu'aucun champ magnétique n'est appliqué au canal (10), le détecteur comprenant en outre une source de lumière laser (16) qui est configurée pour éclairer la particule magnétique (14) restant dans la région de mesure (15) lorsqu'aucun champ magnétique n'est appliqué au canal (10),
dans lequel le canal (10) est configuré de sorte que les particules magnétiques (14) s'étalent d'une manière plane lorsqu'elles se déplacent le long d'un trajet s'élargissant progressivement à l'intérieur du canal de détection jusqu'à ce qu'elles soient capturées dans la région de mesure (15) sous la force magnétique du moyen de génération de champ magnétique (21) ;
le canal (10) ayant une largeur maximale de canal b au niveau de la région de mesure (15) dans une direction perpendiculaire à la direction d'écoulement du canal (10) ;
**caractérisé en ce que**
la largeur a du moyen de génération de champ magnétique (21), qui est la distance entre les surfaces (C, D) n'ayant pas de pôles magnétiques, dans la direction perpendiculaire à la direction d'écoulement du canal (10), et la largeur maximale de canal b du canal (10) au niveau de la région de mesure (15) dans la direction perpendiculaire à la direction d'écoulement du canal (10), satisfont au rapport *a*/*b* de 1,11 ≤ *a*/*b* ≤ 1,85.

2. Détecteur selon la revendication 1, dans lequel la largeur a est supérieure à la largeur *b* d'au moins 0,6 mm.

3. Dispositif d'analyse d'échantillon comprenant le détecteur selon la revendication 1 ou la revendication 2, dans lequel :
le canal (10) est un canal de détection (10) à travers lequel le liquide échantillon contenant la substance spécifique et la particule magnétique (14) liée à la substance spécifique est introduit dans la région de mesure (15) ;
le moyen de génération de champ magnétique (21) est positionné à l'extérieur du canal de détection (10) et au voisinage de la région de mesure (15) de manière à capturer la particule magnétique (14) dans la région de mesure (15) après l'introduction de la particule magnétique (14) dans le canal de détection (10) ; et
le dispositif d'analyse d'échantillon comprend en outre :
un moyen d'alimentation qui alimente le canal de détection (10) en liquide échantillon ;
un moyen de mesure qui mesure la substance spécifique capturée dans la région de mesure (15) ; et
un moyen de décharge qui décharge la particule magnétique (14) hors du canal de détection (10) après la mesure.

4. Dispositif d'analyse d'échantillon selon la revendication 3, dans lequel :
le canal de détection (10) a une entrée (12) à travers laquelle le moyen d'alimentation introduit le liquide échantillon, et une sortie (13) à travers laquelle le moyen de décharge décharge le liquide échantillon après la mesure.
